# EUROPÄISCHE PATENTSCHRIFT

(11) **EP 0 683 780 B1**
(45) Veröffentlichungstag und Bekanntmachung des Hinweises auf die Patenterteilung: **26.04.2000**
(21) Anmeldenummer: 94906909.0
(22) Anmeldetag: 07.02.1994
(51) Int. Cl.: C07D 471/04, A61K 31/435

(54) **IMIDAZOPYRIDINE UND IHRE ANWENDUNG ZUR BEHANDLUNG VON MAGEN-DARM KRANKHEITEN**
IMIDAZOPYRIDINES AND THEIR USE IN TREATING GASTROINTESTINAL DISEASES
IMIDAZOPYRIDINES ET LEUR UTILISATION POUR TRAITER DES MALADIES GASTRO-INTESTINALES

(30) Priorität: 15.02.1993 CH 45393; 29.06.1993 CH 194593
(43) Veröffentlichungstag der Anmeldung: 29.11.1995
(73) Patentinhaber: Byk Gulden Lomberg Chemische Fabrik GmbH, 78467 Konstanz (DE)
(72) Erfinder: SENN-BILFINGER, Jörg, D-78464 Konstanz (DE); GRUNDLER, Gerhard, D-78464 Konstanz (DE); RAINER, Georg, D-78464 Konstanz (DE); POSTIUS, Stefan, D-78467 Konstanz (DE); RIEDEL, Richard, D-88339 Bad Waldsee (DE); SIMON, Wolfgang-Alexander, D-78464 Konstanz (DE)
(86) Internationale Anmeldenummer: EP9400335
(87) Internationale Veröffentlichungsnummer: WO9418199

(56) Entgegenhaltungen:
- EP-A- 0 033 094
- EP-A- 0 268 989
- EP-A- 0 308 917

## Beschreibung

### Anwendungsgebiet der Erfindung

Die Erfindung betrifft Imidazopyridine, die in der pharmazeutischen Industrie als Wirkstoffe für die Herstellung von Arzneimitteln verwendet werden sollen.

### Bekannter technischer Hintergrund

In der europäischen Patentanmeldung EP-A-0 033 094 werden Imidazo[1,2-a]pyridine beschrieben, die in 8-Position einen Arylsubstituenten tragen, der bevorzugt ein Phenyl-, Thienyl-, Pyridyl- oder ein durch Chlor, Fluor, Methyl, tert.-Butyl, Trifluormethyl , Methoxy oder Cyan substituierter Phenylrest ist. Als besonders interessante Arylreste sind in der EP-A-0 033 094 die Reste Phenyl, o- oder p-Fluorphenyl, p-Chlorphenyl und 2,4,6-Trimethylphenyl genannt, wovon die Reste Phenyl, o- oder p-Fluorphenyl und 2,4,6-Trimethylphenyl besonders bevorzugt sind. - In den europäischen Patentanmeldungen EP-A-0 204 285, EP-A-0 228 006, EP-A-0 268 989 und EP-A-0 308 917 werden Imidazo[1,2-a]pyridine beschrieben, die in 3-Position einen ungesättigten aliphatischen Rest, insbesondere einen (substituierten) Alkinylrest tragen. - In der europäischen Patentanmeldung EP-A-0 266 890 werden Imidazo[1,2-a]pyridine beschrieben, die in 8-Position durch einen Alkenyl-, Alkyl- oder Cycloalkylalkylrest substituiert sind.

### Beschreibung der Erfindung

Es wurde nun gefunden, daß die nachfolgend näher beschriebenen Verbindungen, die sich von den Verbindungen des Standes der Technik insbesondere durch die Substitution in 3- oder in 8-Position unterscheiden, überraschende und besonders vorteilhafte Eigenschaften besitzen.

Gegenstand der Erfindungen sind Verbindungen der Formel I (siehe beigefügtes Formelblatt), worin
R0 Methyl oder Hydroxymethyl,
R1 1-4C-Alkyl,
R2 1-4C-Alkyl,
R3 1-4C-Alkoxy und
A 0 (Sauerstoff) oder NH bedeutet,
und ihre Salze.

1-4C-Alkyl steht für geradkettige oder verzweigte Alkylreste mit 1 bis 4 Kohlenstoffatomen. Beispielsweise seien genannt der Butyl-, iso-Butyl-, sec.-Butyl-, tert.-Butyl-, Propyl-, Isopropyl-, Ethyl- und insbesondere der Methylrest.

1-4C-Alkoxy steht für ein Sauerstoffatom, an das einer der vorstehend genannten 1-4C-Alkylreste gebunden ist. Bevorzugt ist der Methoxyrest.

Als Salze kommen für Verbindungen der Formel I bevorzugt alle Säureadditionssalze in Betracht. Besonders erwähnt seien die pharmakologisch verträglichen Salze der in der Galenik üblicherweise verwendeten anorganischen und organischen Säuren. Pharmakologisch unverträgliche Salze, die beispielsweise bei der Herstellung der erfindungsgemäßen Verbindungen im industriellen Maßstab als Verfahrensprodukte zunächst anfallen können, werden durch dem Fachmann bekannte Verfahren in pharmakologisch verträgliche Salze übergeführt. Als solche eignen sich wasserlösliche und wasserunlösliche Säureadditionssalze mit Säuren wie beispielsweise Salzsäure, Bromwasserstoffsäure, Phosphorsäure, Salpetersäure, Schwefelsäure, Essigsäure, Zitronensäure, D-Gluconsäure, Benzoesäure, 2-(4-Hydroxybenzoyl)-benzoesäure, Buttersäure, Sulfosalicylsäure, Maleinsäure, Laurinsäure, Äpfelsäure, Fumarsäure, Bernsteinsäure, Oxalsäure, Weinsäure, Embonsäure, Stearinsäure, Toluolsulfonsäure, Methansulfonsäure oder 3-Hydroxy-2-naphtoesäure, wobei die Säuren bei der Salzherstellung - je nachdem, ob es sich um eine ein- oder mehrbasige Säure handelt und je nachdem, welches Salz gewünscht wird - im äquimolaren oder einem davon abweichenden Mengenverhältnis eingesetzt werden.

Als beispielhafte bevorzugte Verbindungen sind zu nennen die Verbindungen 3-Hydroxymethyl-8-(2-methoxycarbonylamino-6-methyl-benzylamino)-2-methylimidazo[1,2-a]pyridin, 3-Hydroxymethyl-8-(2-methoxycarbonylamino-6-methyl-benzyloxy)-2-methyl-imidazo[1,2-a]pyridin, 8-(2-Methoxycarbonylamino-6-methyl-benzylamino)-2,3-dimethyl-imidazo[1,2-a]pyridin und 8-(2-Methoxy-carbonylamino-6-methyl-benzyloxy)-2,3-dimethyl-imidazo[1,2-a]pyridin und ihre Salze.

Weiterer Gegenstand der Erfindung ist ein Verfahren zur Herstellung der Verbindungen der Formel I und ihrer Salze. Das Verfahren ist dadurch gekennzeichnet, daß man
a) zur Herstellung von Verbindungen der Formel I, in denen R0 Hydroxymethyl bedeutet, Verbindungen der Formel II (siehe das beiliegende Formelblatt), worin R1, R2, R3 und A die oben angegebenen Bedeutungen haben, reduziert, oder daß man
b) zur Herstellung von Verbindungen der Formel I, in denen R0 Methyl bedeutet, Verbindungen der Formel III (siehe das beiliegende Formelblatt), worin R1 und A die oben angegebenen Bedeutungen haben, mit Verbindungen der Formel IV (siehe beiliegendes Formelblatt), worin R2 und R3 die oben angegebenen Bedeutungen haben und X eine geeignete Abgangsgruppe darstellt, umsetzt, oder daß man
c) zur Herstellung von Verbindungen der Formel I, in denen R0 Methyl bedeutet, Verbindungen der Formel V (siehe beiliegendes Formelblatt), worin R1, R2 und A die oben angegebenen Bedeutungen haben, mit Verbindungen der Formel VI (siehe beiliegendes Formelblatt), worin R3 die oben angegebenen Bedeutungen hat und Y eine geeignete Abgangsgruppe darstellt, umsetzt
und daß man gewünschtenfalls anschließend die nach a), b) oder c) erhaltenen Verbindungen I in ihre Salze überführt, oder daß man gewünschtenfalls anschließend aus erhaltenen Salzen der Verbindungen I die Verbindungen I freisetzt.

Die Reduktion der Verbindungen II wird in einer dem Fachmann an sich gewohnten Weise vorgenommen. Sie erfolgt in inerten Lösungsmitteln, z.B. niederen aliphatischen Alkoholen, z.B. unter Verwendung geeigneter Hydride, wie beispielsweise Natriumborhydrid, gewünschtenfalls unter Zusatz von Wasser.

Die Umsetzung der Verbindungen III mit den Verbindungen IV erfolgt auf eine dem Fachmann an sich vertraute Weise, beispielsweise unter analoger Anwendung solcher Verfahren, wie sie in den europäischen Patentanmeldungen EP-A-0 033 094 oder EP-A-0 308 917 beschrieben sind.

Eine geeignete Abgangsgruppe ist beispielsweise ein Halogenatom (bevorzugt Chlor oder Brom) oder eine Methansulfonyloxygruppe. Die Umsetzung erfolgt vorteilhafterweise in Gegenwart einer Base (z.B. eines anorganischen Hydroxids, wie Natriumhydroxid, oder eines anorganischen Carbonates, wie Kaliumcarbonat, oder einer organischen Stickstoffbase, wie Triethylamin, Pyridin, Kollidin oder 4-Dimethylaminopyridin), wobei durch Zusatz von Katalysatoren, wie Alkaliiodid oder Tetrabutylammoniumbromid, die Reaktionsführung begünstigt werden kann.

Die Umsetzung der Verbindungen V mit den Verbindungen VI erfolgt ebenfalls auf eine dem Fachmann an sich bekannte Weise wie sie für die Herstellung von aromatischen Urethanen üblich ist, bevorzugt durch Umsetzung der Verbindungen V mit Halogenameisensäureestern (Y = Halogen), wie Chlorameisensäureestern, in inerten Lösungsmitteln. Die Umsetzung erfolgt vorteilhafterweise in Gegenwart eines säurebindenden Mittels (Protonenakzeptors). Als Protonenakzeptoren seien beispielsweise Alkalimetallcarbonate (wie Kaliumcarbonat) oder -hydrogencarbonate (wie Natriumhydrogencarbonat), oder tertiäre Amine (wie Triethylamin) genannt.

Welche Reaktionsbedingungen für die Durchführung des Verfahrens im einzelnen erforderlich sind, ist dem Fachmann aufgrund seines Fachwissens geläufig.

Die Isolierung und Reinigung der erfindungsgemäßen Substanzen erfolgt in an sich bekannter Weise z.B. derart, daß man das Lösungsmittel im Vakuum abdestilliert und den erhaltenen Rückstand aus einem geeigneten Lösungsmittel umkristallisiert oder einer der üblichen Reinigungsmethoden, wie beispielsweise der Säulenchromatographie an geeignetem Trägermaterial, unterwirft.

Säureadditionssalze erhält man durch Auflösen der freien Base in einem geeigneten Lösungsmittel, z.B. in einem chlorierten Kohlenwasserstoff, wie Methylenchlorid oder Chloroform, einem niederen aliphatischen Alkohol (Ethanol, Isopropanol), einem Keton, wie Aceton, oder einem Ether, wie THF oder Diisopropylether, das die gewünschte Säure enthält, oder dem die gewünschte Säure anschließend zugegeben wird.

Die Salze werden durch Filtrieren, Umfällen, Ausfällen mit einem Nichtlösungsmittel für das Anlagerungssalz oder durch Verdampfen des Lösungsmittels gewonnen. Erhaltene Salze können durch Alkalisierung, z.B. mit wäßriger Ammoniaklösung, in die freien Basen umgewandelt werden, welche wiederum in Säureadditionssalze übergeführt werden können. Auf diese Weise lassen sich pharmakologisch nicht verträgliche Säureadditionssalze in pharmakologisch verträgliche Säureadditionssalze umwandeln.

Die Ausgangsverbindungen II können in an sich bekannter Weise hergestellt werden, beispielsweise durch Umsetzung der Verbindungen VII mit den Verbindungen VIII (siehe beiliegendes Formelblatt), worin R1, R2, R3 und A die oben angegebenen Bedeutungen haben und X eine geeignete Abgangsgruppe, z.B. ein Halogenatom (bevorzugt Chlor oder Brom) darstellt, oder unter analoger Anwendung solcher Verfahren, wie sie beispielsweise in den europäischen Patentanmeldungen EP-A-0 033 094 oder EP-A-0 308 917 beschrieben sind.

Die Ausgangsverbindungen III sind aus der europäischen Patentanmeldung EP-A-0 299 470, die Ausgangsverbindungen IV aus der europäischen Patentanmeldung EP-A-0 308 917 bekannt.

Die Ausgangsverbindungen V können in an sich bekannter Weise aus den entsprechenden Nitroverbindungen durch Reduktion hergestellt werden. Die Nitroverbindungen ihrerseits lassen sich aus den Verbindungen III und entsprechenden, zu den Verbindungen IV korrespondierenden Nitroverbindungen herstellen.

Die folgenden Beispiele dienen der näheren Erläuterung zur Herstellung der erfindungsgemäßen Verbindungen. Insbesondere dienen die Beispiele auch dazu, die Umsetzungen gemäß den Verfahrensvarianten a, b und c sowie die Herstellung ausgewählter Ausgangsverbindungen exemplarisch zu beschreiben. Ebenso können weitere Verbindungen der Formel I sowie weitere Ausgangsverbindungen, deren Herstellung nicht explizit beschrieben ist, in analoger oder in einer dem Fachmann an sich vertrauten Weise unter Anwendung üblicher Verfahrenstechniken hergestellt werden. Die Abkürzung RT steht für Raumtemperatur, h steht für Stunde(n), Schmp. für Schmelzpunkt, Zers. für Zersetzung.

### Beispiele

### 1. 3-Formyl-8-(2-methoxycarbonylamino-6-methylbenzylamino)-2-methyl-imidazol[1,2-a]pyridin

Eine Lösung von 2 g 8-Amino-3-formyl-2-methylimidazo[1,2-a]pyridin in 40 ml trockenem Tetrahydrofuran wird bei RT mit einer Suspension von 400 mg käuflichem 80%-igem Natriumhydrid in 10 ml trockenem Tetrahydrofuran versetzt. Nach kurzzeitiger Erwärmung auf 50°C setzt lebhafte Gasentwicklung ein. Nach beendeter Gasentwicklung wird auf 0°C abgekühlt und eine Lösung von 3,9 g 2-Methoxycarbonylamino-6-methyl-benzylbromid in 40 ml trockenem Tetrahydrofuran eingetropft. Danach wird wiederum auf 50°C erwärmt und die Temperatur 3 h gehalten. Anschließend wird auf Eiswasser gegossen, mit wenig verdünnter Salzsäure neutralisiert und viermal mit Ethylacetat extrahiert. Die gesammelten organischen Phasen werden mit Wasser gewaschen und über Natriumsulfat getrocknet. Das Lösungsmittel wird im Vakuum abgezogen und der dunkelbraune, dickflüssige Rückstand an Kieselgel (Ethylacetat : Petrolether = 1:1 als Elutionsmittel) chromatographiert. Nach Umkristallisation aus Isopropanol werden 2,5 g der Titelverbindung vom Schmp. 188-190°C (Zers.) erhalten.

### 2. 3-Hydroxymethyl-8-(2-methoxycarbonylamino-6-methylbenzylamino)-2-methylimidazo[1,2-a]pyridin

2 g 3-Formyl-8-(2-methoxycarbonylamino-6-methylbenzylamino)-2-methylimidazo[1,2-a]pyridin werden bei RT in 30 ml Methanol suspendiert, portionsweise mit 0,2 g Natriumborhydrid versetzt und 1 h bei RT gerührt. Anschließend wird die Hälfte des Lösungsmittels im Vakuum abgezogen, der Rückstand auf Eiswasser gegossen, mit einigen Tropfen verdünnter Salzsäure neutralisiert und viermal mit Ethylacetat extrahiert. Die gesammelten organischen Phasen werden mit Wasser gewaschen und über Natriumsulfat getrocknet. Das Lösungsmittel wird im Vakuum abgezogen. Der verbleibende gelbliche Rückstand kristallisiert nach einiger Zeit durch. Nach Umkristallisation aus Ethylacetat werden 1,3 g der Titelverbindung vom Schmp. 170-172°C erhalten.

### 3. 3-Formyl-8-(2-methoxycarbonylamino-6-methylbenzyloxy)-2-methylimidazo[1,2-a]pyridin

2,6 9 3-Formyl-8-hydroxy-2-methylimidazo[1,2-a]pyridin werden unter Feuchtigkeitsausschluß bei 50°C in 50 ml trockenem Acetonitril gelöst und nach Abkühlen auf RT mit 2,8 g käuflichem Kaliumfluorid (50 Gew.-%) auf Kieselgur (z.B. Celite®) versetzt. Eine Lösung von 3,6 g 2-Methoxycarbonylamino-6-methylbenzylbromid in 50 ml trockenem Acetonitril wird eingetropft und das Gemisch 6 h bei 70°C erwärmt. Nach Abkühlen auf RT wird auf Eiswasser gegossen, mit wenigen Tropfen 6 N Natronlauge auf pH 9 gestellt und mit Ethylacetat extrahiert. Die gesammelten organischen Phasen werden mit Wasser gewaschen und über Natriumsulfat getrocknet. Nach Abziehen des Lösungsmittels im Vakuum und Verrühren in wenig kaltem Methanol werden 3,2 g der Titelverbindung vom Schmp. 196-198°C erhalten.

### 4. 3-Hydroxymethyl-8-(2-methoxycarbonylamino-6-methylbenzyloxy)-2-methylimidazo[1,2-a]pyridin

Analog Beispiel 2 werden 2,1 g der Titelverbindung vom Schmp. 185-187°C aus 3 g 3-Formyl -8-(2-methoxycarbonylamino-6-methylbenzyloxy)-2-methylimidazo[1,2-a]pyridin und 400 mg Natriumborhydrid erhalten.

### 5. 8-(2-Methoxycarbonylamino-6-methylbenzylamino)-2,3-dimethylimidazo-[1,2-a]pyridin

Eine Lösung von 4,03 g 8-Amino-2,3-dimethylimidazo[1,2-a]pyridin und 6,41 g 2-Methoxycarbonylamino-6-methylbenzylchlorid in 400 ml wasserfreiem Aceton wird mit 4,5 g Natriumiodid und 6,63 g wasserfreiem Natriumcarbonat versetzt und anschließend 6 h am Rückfluß gekocht. Nach Abkühlen auf RT gibt man 400 ml Wasser zu und destilliert das Aceton im Wasserstrahlvakuum ab. Der wässerige Rückstand wird dann mit 3 x 200 ml Essigester extrahiert. Die vereinigten organischen Extrakte werden mit 300 ml Wasser gewaschen, über Magnesiumsulfat getrocknet und eingeengt. Der Rückstand wird durch Chromatographie an Kieselgel (Fließmittel: Toluol/Dioxan = 9 : 1) gereinigt. Die Fraktionen mit Rf = 0,2 werden eingeengt und anschließend aus Diisopropylether kristallisiert. 4,71 g (56%) der Titelverbindung werden isoliert. Schmp.: 136-138°C.
a) Durch Umsetzung der in Aceton gelösten Titelverbindung mit 12 N Salzsäure erhält man das Hydrochlorid der Titelverbindung vom Schmp. 211-212°C (Zers.).
b) Durch Umsetzung der in Tetrahydrofuran gelösten Titelverbindung mit Methansulfonsäure erhält man das Methansulfonat der Titelverbindung vom Schmp. 181-182°C (Zers.).
c) Durch Umsetzung der in Aceton gelösten Titelverbindung mit Fumarsäure erhält man das Hemifumarat der Titelverbindung vom Schmp. 191-192°C (Zers.).

### 6. 8-(2-Methoxycarbonylamino-6-methylbenzyloxy)-2,3-dimethylimidazo-[1,2-a]pyridin

Eine Suspension von 7,2 g 8-Hydroxy-2,3-dimethylimidazo[1,2-a]pyridin in 130 ml trockenem Acetonitril wird bei RT mit 8 g käuflichem Kaliumfluorid (50 Gew.-%) auf Kieselgur (z.B. Celite®) versetzt und eine Lösung von 9,5 g 2-Methoxycarbonylamino-6-methyl-benzylchlorid in 150 ml trockenem Acetonitril eingetropft. Das Gemisch wird 9 h bei 70°C erwärmt, nach Abkühlen auf RT auf 1 l Eiswasser gegossen. 3 x mit Ethylacetat extrahiert und die gesammelten organischen Phasen nach Waschen mit destilliertem Wasser über Natriumsulfat getrocknet. Nach Abziehen des organischen Lösungsmittels im Vakuum wird der verbleibende Rückstand in wenig Ethylacetat ausgerührt, der sich abscheidende Feststoff abfiltriert, mit wenig Ethylacetat und Ether nachgewaschen und getrocknet. Nach Umkristallisation aus Isopropanol werden 2,2 g der Titelverbindung vom Schmp. 176-177°C erhalten.

### 7. 8-(6-Methyl-2-nitrobenzylamino)-2,3-dimethylimidazo[1,2-a]pyridin

Zu einer Lösung von 8-Amino-2,3-dimethylimidazo[1,2-a]pyridine (14,7 g) und 6-Methyl-2-nitrobenzylchlorid (18,6 g) in 100 ml Aceton (100 ml) gibt man bei RT 15,0 g Natriumiodid und 31,0 g Natriumcarbonat und erhitzt anschließend für 6 h unter Rückfluß zum Sieden. Nach Abkühlen der Lösung auf RT und Einengen wird der Rückstand in einem Gemisch aus 200 ml Ethylacetat und 200 ml Wasser gelöst und die organische Phase wird abgetrennt. Nach drei weiteren Extraktionen mit jeweils 100 ml Ethylacetat werden die vereinigten organischen Phasen über Magnesiumsulfat getrocknet und anschliessend auf 80 ml eingeengt. 12,1 g der Titelverbindung kristallisieren als schwach gelber Feststoff. Die Mutterlauge wird eingeengt. Nach chromatografischer Reinigung des Rückstandes an Kieselgel (Fließmittel: Toluol/Dioxan = 6:1) erhält man weitere 14 g des kristallinen Produkts. Nach Umkristallisation beider Fraktionen aus Ethylacetat erhält man 21,5 g (76 %) der Titelverbindung vom Schmp. 160-162°C.

### 8. 8-(2-tert-Butoxycarbonylamino-6-methylbenzylamino)-2,3-dimethylimidazo[1,2-a]pyridin

Ausgehend von 8-Amino-2,3-dimethylimidazo[1,2-a]pyridin (4,8 g), 2-tert-Butoxycarbonylamino-6-methylbenzylchlorid (9,2 g), Natriumiodid (5,5 g) und Natriumcarbonat (8,0 g) in Aceton (250 ml) erhält man in analoger Anwendung des Verfahrens von Beispiel 7 nach Chromatografie an Kieselgel (Fließmittel Toluol/Dioxan 20:1) und Umkristallisation aus Diisopropylether 7,1 g (62%) der Titelverbindung vom Schmp. 149-152°C.

### 9. 8-(2-tert-Butoxycarbonylamino-6-methylbenzyloxy)-2,3-dimethylimidazo-[1,2-a]pyridin

Ausgehend von 2,3-Dimethyl-8-hydroxy-imidazo[1,2-a]pyridine (1,6 g), 2-tert-Butoxycarbonylamino-6-methylbenzylchlorid (3,1 g), Natriumiodid (1,8 g) und Natriumcarbonat (2,7 g) in Aceton (350 ml) erhält man in analoger Anwendung des Verfahrens von Beispiel 7 nach Chromatografie an Kieselgel (Fließmittel Toluol/Dioxan 5:1) und Umkristallisation aus Cyclohexan 3,0 g (78%) der Titelverbindung vom Schmp. 128-131°C.

### 10. 8-(2-tert-Butoxycarbonylamino-6-methylbenzylamino)-3-formyl-2-methylimidazo[1,2-a]pyridin

Ausgehend von 8-Amino-3-formyl-2-methylimidazo[1,2-a]pyridin (4,0 g), 2-tert-Butoxycarbonylamino-6-methylbenzylchlorid (7,0 g), Natriumiodid (4,1 g) und Natriumcarbonat (6,1 g) in Aceton (250 ml) erhält man in analoger Anwendung des Verfahrens von Beispiel 7 nach Chromatografie an Kieselgel (Fließmittel Toluol/Dioxan 9:1) und Umkristallisation aus Diisopropylether 7,3 g (81%) der Titelverbindung vom Schmp. 210-212°C.

### 11. 8-(2-tert-Butoxycarbonylamino-6-methylbenzyloxy)-3-formyl-2-methylimidazo[1,2-a]pyridin

a) 4,77 g (0,02 mol) 8-Benzyloxy-2-methylimidazo[1,2-a]pyridin werden in einer Vilsmeier-Mischung aus 20 ml Dimethylformamid und 2,3 ml Phosphoroxychlorid 2,5 h bei 60°C gerührt und in üblicher Weise mit Eis/Wasser und Kaliumhydrogencarbonat aufgearbeitet. Man erhält 8-Benzyloxy-2-methylimidazo[1,2-a]pyridin-3-carboxaldehyd vom Schmp. 105-106°C (aus Diisopropylether). Diese Verbindung wird analog Kaminski et al., J. Med. Chem. 28, 876 (1985), Methode H, zur Titelverbindung vom Schmp. 251-252°C debenzyliert.
b) Ausgehend von 3-Formyl-8-hydroxy-2-methylimidazo[1,2-a]pyridin (2,4 g), 2-tert-Butoxycarbonylamino-6-methylbenzylchlorid (4,2 g), Natriumiodid (2,5 g) und Natriumcarbonat (3,7 g) in Aceton (400 ml) erhält man in analoger Anwendung des Verfahrens von Beispiel 7 nach Umkristallisation aus Diisopropylether/Ethylacetat 4,4 g (80%) der Titelverbindung vom Schmp. 189-191°C.

### 12. 8-(2-Amino-6-methylbenzylamino)-2,3-dimethylimidazo[1,2-a]pyridin

### Methode A:

Eine Lösung von 8-(6-Methyl-2-nitrobenzylamino)-2,3-dimethylimidazo[1,2-a]-pyridin (61 g) in Methanol (5,5 l) wird in Gegenwart von 15 g Palladium auf Aktivkohle (5%) als Katalysator bei RT und unter Atmosphärendruck für 1,5 h hydriert. Nach Abfiltrieren des Katalysators und Einengen wird der Rückstand in siedendem Ethylacetat gelöst (2,7 l). Nach dem Abkühlen auf RT werden 51 g (82%) der Titelverbindung vom Schmp. 206-208°C isoliert.

### Methode B:

6,7 g 8-(2-tert-Butoxycarbonylamino-6-methylbenzylamino)-2,3-dimethylimidazo[1,2-a]pyridin werden bei 25-30°C portionsweise zu einer Mischung aus Trifluoressigsäure (30 ml) und Anisol (3 ml) zugefügt. Nach 30-minütigem Rühren bei RT wird die Lösung in 100 ml Eiswasser gegossen und anschließend mit 75 ml 6N Natronlauge versetzt. Der Niederschlag wird abfiltriert und an Kieselgel chromatografisch gereinigt (Lösungsmittel: Toluol/Dioxan = 8:1). Nach dem Umkristallisieren aus Ethylacetat erhält man 3,1 g (62%) der Titelverbindung vom Schmp. 206-208°C.

### 13. 8-(2-Amino-6-methylbenzyloxyl-3-formyl-2-methylimidazo[1,2-a]pyridin

Ausgehend von 8-(2-tert-Butoxycarbonylamino-6-methylbenzyloxy)-3-formyl-2-methylimidazo[1,2-a]pyridin (5,0 g) und Trifluoressigsäure (40 ml) erhält man in analoger Anwendung des Verfahrens von Beispiel 12 (Methode B) 3,57 g (96%) der Titeiverbindung vom Schmp. 144-150°C (Zers.).

### 14. 8-(2-Ethoxycarbonylamino-6-methylbenzylamino)-2,3-dimethylimidazo[1,2-a]pyridin

Eine Lösung von 0,65 g Chlorameisensäureethylester gelöst in 10 ml Dichlormethan wird tropfenweise zu einer Lösung von 0,98 g 8-(2-Amino-6-methylbenzylamino)-2,3-dimethylimidazo[1,2-a]pyridin in 50 ml Dichlormethan hinzugefügt. Nach 18-stündigem Rühren bei RT wird die Lösung mit 40 ml gesättigter Natriumbicarbonatlösung extrahiert, mit 40 ml Wasser gewaschen und eingeengt. Der Rückstand wird aus Ethylacetat/Diisopropylether umkristallisiert. Man erhält 0,32 g (26%) der Titelverbindung vom Schmp. 208-210°C (Zers.).

### 15. 8-(2-Isobutoxycarbonylamino-6-methylbenzylamino)-2,3-dimethylimidazo[1,2-a]pyridin

In analoger Anwendung des Verfahrens von Beispiel 14 erhält man ausgehend von 0,56 g 8-(2-Amino-6-methylbenzylamino)-2,3-dimethylimidazo[1,2-a]pyridin in 50 ml Dichlormethan und 0,3 g Chlorameisensäureisobutylester 0,22 g (29%) der Titelverbindung vom Schmp. 144-146°C.

### 16. 8-(2-Isopropoxycarbonylamino-6-methylbenzylamino)-2,3-dimethylimidazo-[1,2-a]pyridin

In analoger Anwendung des Verfahrens von Beispiel 14 erhält man ausgehend von 0,98 g 8-(2-Amino-6-methylbenzylamino)-2,3-dimethylimidazo[1,2-a]pyridin in 50 ml Dichlormethan und 1,5 g Chlorameisensäureisopropylester 0,32 g (25%) der Titelverbindung.

### 17. 8-(2-tert-Butoxycarbonylamino-6-methylbenzylamino)-3-hydroxymethyl-2-methyl imidazo[1,2-a]pyridin

In analoger Anwendung des Verfahrens von Beispiel 2 erhält man ausgehend von 0,15 g 8-(2-tert-Butoxycarbonylamino-6-methylbenzylamino)-3-formyl-2-methylimidazo[1,2-a]pyridin und 15 mg Natriumborhydrid in Methanol 0,12 g der Titelverbindung vom Schmp. 102-104°C.

### 18. 8-(2-tert-Butoxycarbonylamino-6-methylbenzyloxy)-3-hydroxymethyl-2-methylimidazo[1,2-a]pyridin

In analoger Anwendung des Verfahrens von Beispiel 2 erhält man ausgehend von 0,20 g 8-(2-tert-Butoxycarbonylamino-6-methylbenzyloxy)-3-formyl-2-methylimidazo[1,2-a]pyridin und 19 mg Natriumborhydrid in Methanol 0,17 g der Titelverbindung vom Schmp. 140-142°C.

### Gewerbliche Anwendbarkeit

Die Verbindungen der Formel I und ihre Salze besitzen wertvolle pharmakologische Eigenschaften, die sie gewerblich verwertbar machen. Sie weisen insbesondere eine ausgeprägte Magensäuresekretionshemmung und eine ausgezeichnete Magen- und Darmschutzwirkung bei Warmblütern auf. Hierbei zeichnen sich die erfindungsgemäßen Verbindungen durch eine hohe Wirkungsselektivität, eine vergleichsweise lange Wirkungsdauer, eine gute enterale Wirksamkeit, das Fehlen wesentlicher Nebenwirkungen und eine große therapeutische Breite aus.

Unter "Magen- und Darmschutz" wird in diesem Zusammenhang die Verhütung und Behandlung gastrointestinaler Krankheiten, insbesondere gastraintestinaler entzündlicher Krankheiten und Läsionen (wie z.B. Ulcus ventriculi, Ulcus duodeni, Gastritis, hyperazider oder medikamentös bedingter Reizmagen) verstanden, die beispielsweise durch Mikroorganismen (z.B. Helicobacter pylori), Bakterientoxine, Medikamente (z.B. bestimmte Antiphlogistika und Antirheumatika), Chemikalien (z.B. Ethanol), Magensäure oder Streßsituationen verursacht werden können.

In ihren ausgezeichneten Eigenschaften erweisen sich die erfindungsgemäßen Verbindungen an verschiedenen Modellen, in denen die antiulcerogenen und die antisekretorischen Eigenschaften bestimmt werden, überraschenderweise den aus dem Stand der Technik bekannten Verbindungen deutlich überlegen. Aufgrund dieser Eigenschaften sind die Verbindungen der Formel I und ihre pharmakologisch verträglichen Salze für den Einsatz in der Human- und Veterinärmedizin hervorragend geeignet, wobei sie insbesondere zur Behandlung und/oder Prophylaxe von Erkrankungen des Magens und/oder Darms verwendet werden.

Ein weiterer Gegenstand der Erfindung sind daher die erfindungsgemäßen Verbindungen zur Anwendung bei der Behandlung und/oder Prophylaxe der vorstehend genannten Krankheiten.

Ebenso umfaßt die Erfindung die Verwendung der erfindungsgemäßen Verbindungen zur Herstellung von Arzneimitteln, die zur Behandlung und/oder Prophylaxe der vorstehend genannten Krankheiten eingesetzt werden.

Weiterhin umfaßt die Erfindung die Verwendung der erfindungsgemäßen Verbindungen zur Behandlung und/oder Prophylaxe der vorstehend genannten Krankheiten.

Ein weiterer Gegenstand der Erfindung sind Arzneimittel, die ein oder mehrere Verbindungen der Formel I und/oder ihre pharmakologisch verträglichen Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoffe) entweder als solche, oder vorzugsweise in Kombination mit geeigneten pharmazeutischen Hilfs- oder Trägerstoffen in Form von Tabletten, Dragees, Kapseln, Suppositorien, Pflastern (z.B. als TTS), Emulsionen, Suspensionen oder Lösungen eingesetzt, wobei der Wirkstoffgehalt vorteilhafterweise zwischen 0,1 und 95 % beträgt und wobei durch die entsprechende Wahl der Hilfs- und Trägerstoffe eine auf den Wirkstoff und/oder auf den gewünschten Wirkungseintritt genau angepaßte galenische Darreichungsform (z.B. eine Retardform oder eine magensaftresistente Form) erzielt werden kann.

Welche Hilfs- bzw. Trägerstoffe für die gewünschten Arzneimittelformulierungen geeignet sind, ist dem Fachmann aufgrund seines Fachwissens geläufig. Neben Lösemitteln, Gelbildnern, Suppositoriengrundlagen, Tablettenhilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler, Farbstoffe oder insbesondere Permeationspromotoren und Komplexbildner (z.B. Cyclodextrine) verwendet werden.

Die Wirkstoffe können oral, parenteral oder percutan appliziert werden.

Im allgemeinen hat es sich in der Humanmedizin als vorteilhaft erwiesen, den oder die Wirkstoffe bei oraler Gabe in einer Tagesdosis von etwa 0,01 bis etwa 20, vorzugsweise 0,05 bis 5, insbesondere 0,1 bis 1,5 mg/kg Körpergewicht, gegebenenfalls in Form mehrerer, vorzugsweise 1 bis 4 Einzelgaben zur Erzielung des gewünschten Ergebnisses zu verabreichen. Bei einer parenteralen Behandlung können ähnliche bzw. (insbesondere bei der intravenösen Verabreichung der Wirkstoffe) in der Regel niedrigere Dosierungen zur Anwendung kommen. Die Festlegung der jeweils erforderlichen optimalen Dosierung und Applikationsart der Wirkstoffe kann durch jeden Fachmann aufgrund seines Fachwissens leicht erfolgen.

Sollen die erfindungsgemäßen Verbindungen und/oder Salze zur Behandlung der oben genannten Krankheiten eingesetzt werden, so können die pharmazeutischen Zubereitungen auch einen oder mehrere pharmakologisch aktive Bestandteile anderer Arzneimittelgruppen, wie Antacida, beispielsweise Aluminiumhydroxyd, Magnesiumaluminat; Tranquilizer, wie Benzodiazepine, beispielsweise Diazepam; Spasmolytika, wie z.B. Bietamiverin, Camylofin, Anticholinergica, wie z.B. Oxyphencyclimin, Phencarbamid; Lokalanaesthetika, wie z.B. Tetracain, Procain; gegebenenfalls auch Fermente, Vitamine oder Aminosäuren enthalten.

Hervorzuheben ist in diesem Zusammenhang insbesondere die Kombination der erfindungsgemäßen Verbindungen mit Pharmaka, die die Säuresekretion hemmen, wie beispielsweise H₂-Blockern (z.B. Cimetidin, Ranitidin), H⁺/K⁺-ATPase-Hemmstoffen (z.B. Omeprazol, Pantoprazol), oder ferner mit sogenannten peripheren Anticholinergika (z.B. Pirenzepin, Telenzepin) sowie mit Gastrin-Antagonisten mit dem Ziel, die Hauptwirkung in additivem oder überadditivem Sinn zu verstärken und/oder die Nebenwirkungen zu eliminieren oder zu verringern, oder ferner die Kombination mit antibakteriell wirksamen Substanzen (wie z.B. Cephalosporinen, Tetracyclinen, Nalidixinsäure, Penicillinen oder auch Wismutsalzen) zur Bekämpfung von Helicobacter pylori.

### Pharmakologie

Die ausgezeichnete Magenschutzwirkung und die magensäuresekretionshemmende Wirkung der erfindungsgemäßen Verbindungen kann in Untersuchungen an tierexperimentellen Modellen nachgewiesen werden. Die in dem nachstehend aufgeführten Modell untersuchten erfindungsgemäßen Verbindungen sind mit Nummern versehen worden, die den Nummern dieser Verbindungen in den Beispielen entsprechen.

### Prüfung der sekretionshemmenden Wirkung am perfundierten Rattenmagen

In der folgenden Tabelle 1 ist der Einfluß der erfindungsgemäßen Verbindungen nach intraduodenaler Gabe auf die durch Pentagastrin stimulierte Säuresekretion des perfundierten Rattenmagens in vivo dargestellt.

**Tabelle 1**

| Nr. | Dosis (µmol/kg) i .d. | Hemmung der Säureausscheidung (%) |
|---|---|---|
| 2 | 6 | 100 |
| 4 | 10 | 100 |
| 5 | 3 | 100 |
| 6 | 3 | 100 |

### Methodik

Narkotisierten Ratten (CD-Ratte, weiblich, 200-250 g; 1,5 g/kg i.m. Urethan) wurde nach Tracheotomie das Abdomen durch einen medianen Oberbauchschnitt eröffnet und ein PVC-Katheter transoral im Ösophagus sowie ein weiterer via Pylorus derart fixiert, daß die Schlauchenden eben noch in das Magenlumen hineinragten. Der aus dem Pylorus führende Katheter führte über eine seitliche Öffnung in der rechten Bauchwand nach außen.

Nach gründlicher Spülung (ca. 50-100 ml) wurde der Magen mit 37°C warmer physiologischer NaCl-Lösung kontinuierlich durchströmt (0,5 ml/min, pH 6,8-6,9; Brun-Unita I). In dem jeweils im 15 Min.-Abstand aufgefangenen (25 ml Meßzylinder) Effluat wurde der pH-Wert (pH-Meter 632, Glaselektrode EA 147; φ = 5 mm, Metrohm) sowie durch Titration mit einer frisch zubereiteten 0,01 N NaOH bis pH 7 (Dosimat 655 Metrohm) die sezernierte HCl bestimmt.

Die Stimulation der Magensekretion erfolgte durch Dauerinfusion von 1 µg/kg (= 1,65 ml/h) i.v. Pentagastrin (V. fem. sin.) ca. 30 Min. nach Operationsende (d.h. nach Bestimmung von 2 Vorfraktionen). Die zu prüfenden Substanzen wurden intraduodenal in 1 ml/kg Flüssigkeitsvolumen 60 Min. nach Beginn der Pentagastrin-Dauerinfusion verabreicht.

Die Körpertemperatur der Tiere wurde durch Infrarot-Bestrahlung und Heizkissen (automatische, stufenlose Regelung über rektalen Temperaturfühler) auf konstant 37,8 - 38°C gehalten.

In der Tabelle ist diejenige Dosis angegeben, die zu einer maximalen Hemmung der Säuresekretion um 100% führte.

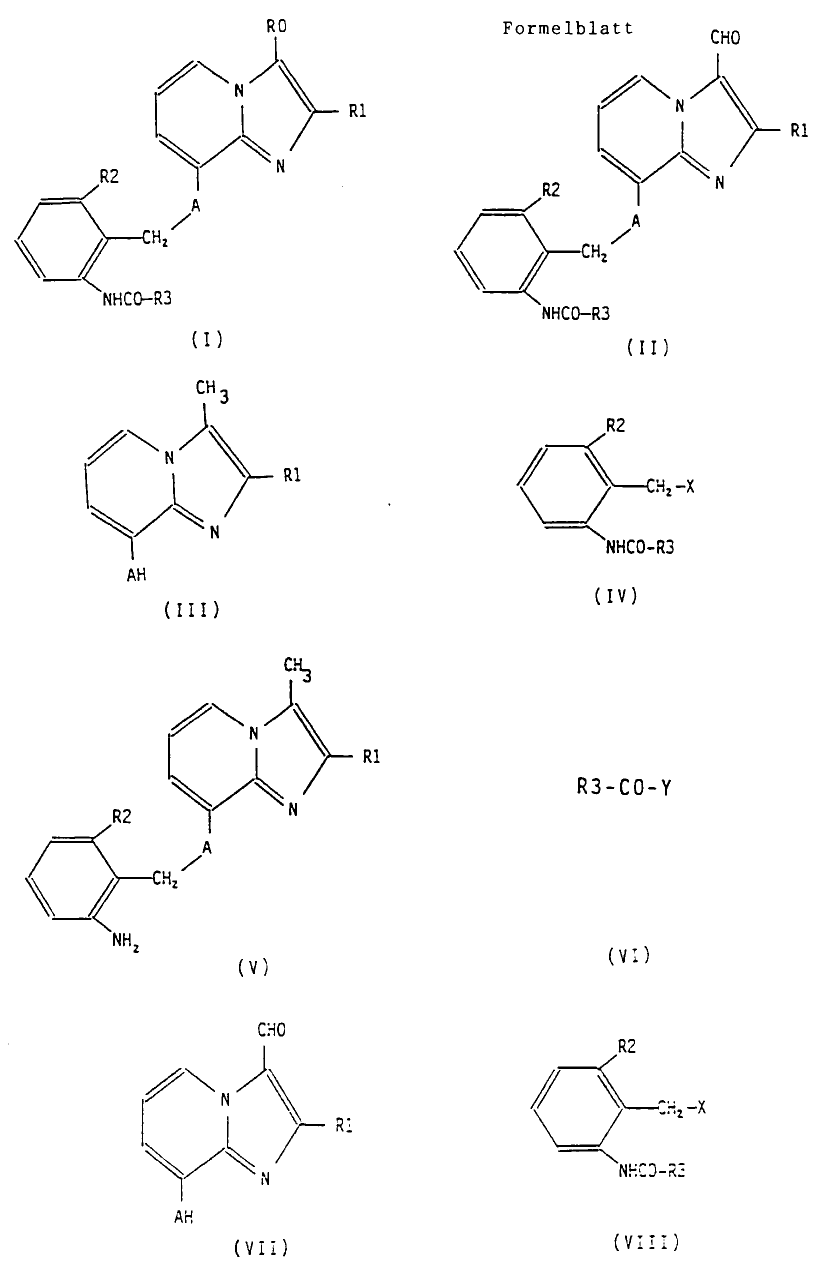

## Patentansprüche

1. Verbindungen der Formel I, 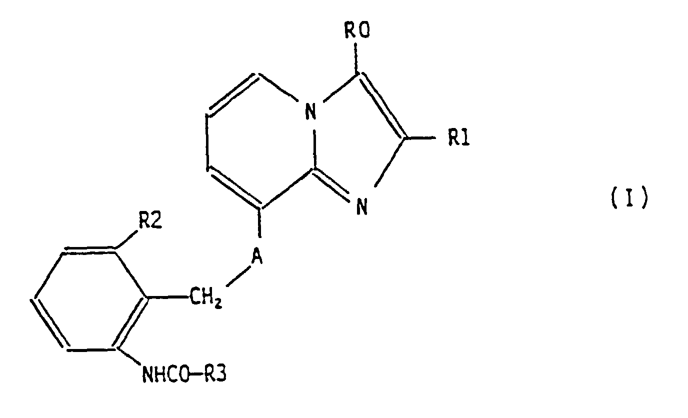 worin
R0 Methyl oder Hydroxymethyl,
R1 1-4C-Alkyl,
R2 1-4C-Alkyl,
R3 1-4C-Alkoxy und
A 0 (Sauerstoff) oder NH bedeutet,
und ihre Salze.

2. Verbindungen der Formel I nach Anspruch 1, worin R0 Methyl bedeutet.

3. Verbindungen der Formel I nach Anspruch 1, worin R0 Hydroxymethyl bedeutet.

4. Verbindung nach Anspruch 1 ausgewählt aus der Gruppe bestehend aus
3-Hydroxymethyl-8-(2-methoxycarbonylamino-6-methylbenzylamino)-2-methylimidazo[1,2-a]pyridin,
3-Hydroxymethyl-8-(2-methoxycarbonylamino-6-methylbenzyloxy)-2-methylimidazo[1,2-a]pyridin,
8-(2-Methoxycarbony]amino-6-methylbenzyloxy)-2,3-dimethylimidazo[1,2-a]-pyridin,
8-(2-tert-Butoxycarbonylamino-6-methylbenzylamirio)-2,3-dimethylimidazo-[1,2-a]pyridin,
8-(2-tert-Butoxycarbonylamino-6-methylbenzyloxy)-2,3-dimethylimidazo[1,2-a]pyridin,
8-(2-Ethoxycarbonylamino-6-methylbenzylamino)-2,3-dimethylimidazo[1,2-a]-pyridin,
8-(2-Isobutoxycarbonylamino-6-methylbenzylamino)-2,3-dimethylimidazo[1,2-a]pyridin,
8-(2-Isopropoxycarbonylamino-6-methylbenzylamino)-2,3-dimethylimidazo-[1,2-a]pyridin,
8-(2-tert-Butoxycarbonylamino-6-methylbenzylamino)-3-hydroxymethyl-2-methylimidazo[1,2-a]pyridin und
8-(2-tert-Butoxycarbonylamino-6-methylbenzyloxy)-3-hydroxymethyl-2-methylimidazo[1,2-a]pyridin
oder ein Salz davon.

5. Verbindung nach Anspruch 1 mit der Bezeichnung
8-(2-Methoxycarbonylamino-6-methylbenzylamino)-2,3-dimethylimidazo[1,2-a]pyridin, oder ein Salz davon.

6. Verfahren zur Herstellung der Verbindungen der Formel I nach Anspruch 1 und ihrer Salze, dadurch gekennzeichnet, daß man
a) zur Herstellung von Verbindungen der Formel I, in denen R0 Hydroxymethyl bedeutet, Verbindungen der Formel II 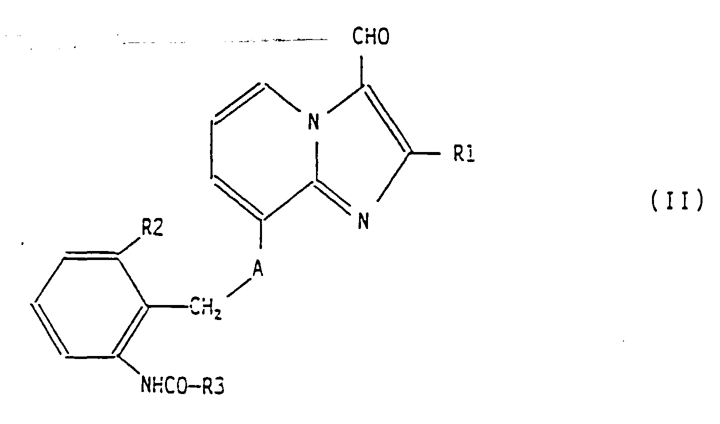 worin R1, R2, R3 und A die in Anspruch 1 angegebenen Bedeutungen haben, reduziert, oder daß man
b) zur Herstellung von Verbindungen der Formel I, in denen R0 Methyl bedeutet, Verbindungen der Formel III 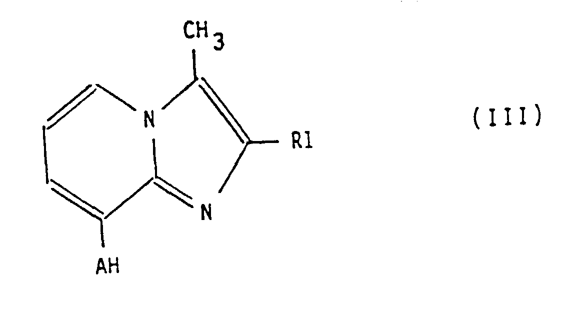 worin R1 und A die in Anspruch 1 angegebenen Bedeutungen haben, mit Verbindungen der Formel IV 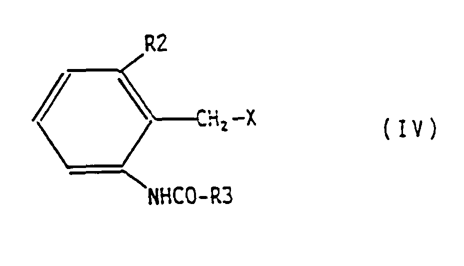 worin R2 und R3 die in Anspruch 1 angegebenen Bedeutungen haben und X eine geeignete Abgangsgruppe darstellt, umsetzt, oder daß man
c) zur Herstellung von Verbindungen der Formel I, in denen R0 Methyl bedeutet, Verbindungen der Formel V 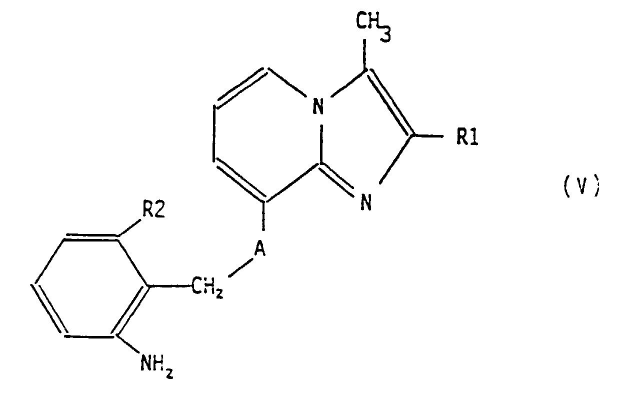 worin R1, R2 und A die in Anspruch 1 angegebenen Bedeutungen haben, mit Verbindungen der Formel VI
R3-CO-Y (VI)
worin R3 die in Anspruch 1 angegebenen Bedeutungen hat und Y eine geeignete Abgangsgruppe darstellt, umsetzt
und daß man gewünschtenfalls anschließend die nach a), b) oder c) erhaltenen Verbindungen I in ihre Salze überführt, oder daß man gewünschtenfalls anschließend aus erhaltenen Salzen der Verbindungen I die Verbindungen I freisetzt.

7. Arzneimittel enthaltend eine Verbindung nach Anspruch 1 und/oder ein pharmakologisch verträgliches Salz davon.

8. Verbindungen nach Anspruch 1 und ihre pharmakologisch verträglichen Salze zur Anwendung bei der Verhütung und Behandlung gastrointestinaler Krankheiten.

9. Verwendung von Verbindungen nach Anspruch 1 und ihren pharmakologisch verträglichen Salzen zur Herstellung von Arzneimitteln für die Verhütung und Behandlung gastrointestinaler Krankheiten.

## Claims

1. Compounds of the formula I 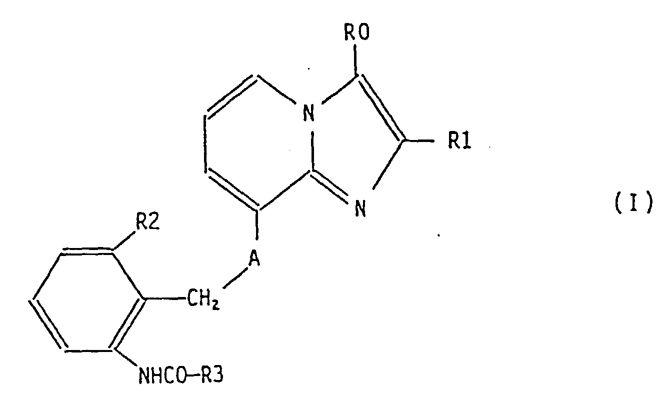 in which
R0 denotes methyl or hydroxymethyl,
R1 denotes 1-4C-alkyl,
R2 denotes 1-4C-alkyl,
R3 denotes 1-4C-alkoxy and
A denotes 0 (oxygen) or NH,
and the salts thereof.

2. Compounds of the formula I according to claim 1, wherein R0 denotes methyl.

3. Compounds of the formula I according to claim 1, wherein R0 denotes hydroxymethyl.

4. Compound according to claim 1 selected from the group comprising
3-Hydroxymethyl-8-(2-methoxycarbonylamino-6-methylbenzylamino)-2-methyl-imidazo[1,2-a]pyridine,
3-Hydroxymethyl-8-(2-methoxycarbonylamino-6-methylbenzyloxy)-2-methylimidazo[1,2-a]pyridine,
8-(2-Methoxycarbonylamino-6-methylbenzyloxy)-2,3-dimethylimidazo[1,2-a]-pyridine,
8-(2-tert-Butoxycarbonylamino-6-methylbenzylamino)-2,3-dimethylimidazo-[1,2-a]pyridine,
8-(2-tert-Butoxycarbonylamino-6-methylbenzyloxy)-2,3-dimethylimidazo-[1,2-a]pyridine,
8-(2-Ethoxycarbonylamino-6-methylbenzylamino)-2,3-dimethylimidazo[1,2-a]-pyridine,
8-(2-Isobutoxycarbonylamino-6-methylbenzylamino)-2,3-dimethylimidazo-[1,2-a]pyridine,
8-(2-Isopropoxycarbonylamino-6-methylbenzylamino)-2,3-dimethylimidazo-[1,2-a]pyridine,
8-(2-tert-Butoxycarbonylamino-6-methylbenzylamino)-3-hydroxymethyl-2-methylimidazo[1,2-a]pyridine and
8-(2-tert-Butoxycarbonylamino-6-methylbenzyloxy)-3-hydroxymethyl-2-methyl-imidazo[1,2-a]pyridine
or a salt thereof.

5. Compound according to claim 1 named 8-(2-Methoxycarbonylamino-6-methylbenzylamino)-2,3-dimethylimidazo-[1,2-a]pyridine, or a salt thereof.

6. Process for the preparation of the compounds of the formula I as claimed in claim 1 and the salts thereof, which comprises
a) for the preparation of the compounds of the formula I, in which R0 denotes hydroxymethyl, reducing compounds of the formula II 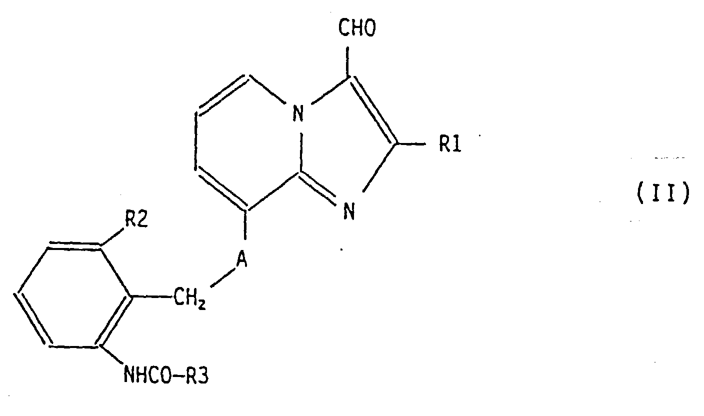 in which R1, R2, R3 and A have the meanings given in claim 1, or comprises
b) for the preparation of the compounds of the formula I, in which R0 denotes methyl, reacting compounds of the formula III 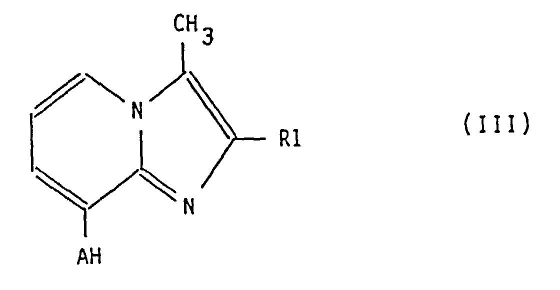 in which R1 and A have the meanings given in claim 1, with compounds of the formula IV 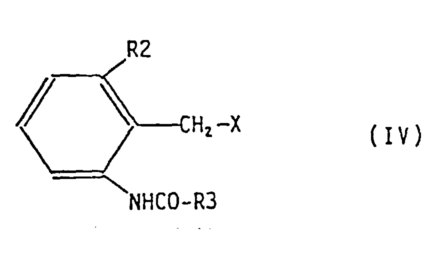 in which R2 and R3 have the meanings given in claim 1 and X denotes a suitable leaving group, or comprises
c) for the preparation of the compounds of the formula I, in which R0 denotes methyl, reacting compounds of the formula V 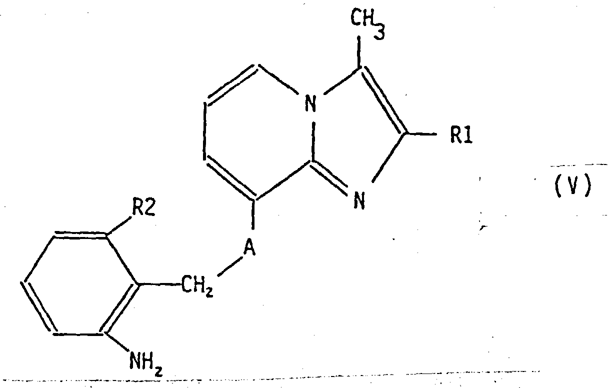 in which R1, R2 and A have the meanings given in claim 1, with compounds of the formula VI
R3-CO-Y (VI)
wherein R3 has the meanings given in claim 1 and Y denotes a suitable leaving group,
and, if desired, subsequently converting the resulting compounds I obtained according to a), b) or c) into the salts thereof, or comprises, if desired, subsequently liberating the compounds I from the resulting salts of the compounds I.

7. A pharmaceutical containing a compound as claimed in claim 1 and/or a pharmacologically compatible salt thereof.

8. A compound as claimed in claim 1 and the pharmacologically compatible salts thereof for use for the prevention and treatment of gastrointestinal disorders.

9. The use of compounds according to claim 1 and the pharmacologically compatible salts thereof for the production of pharmaceuticals for the prevention and treatment of gastrointestinal disorders.

## Revendications

1. Composés de formule (I), 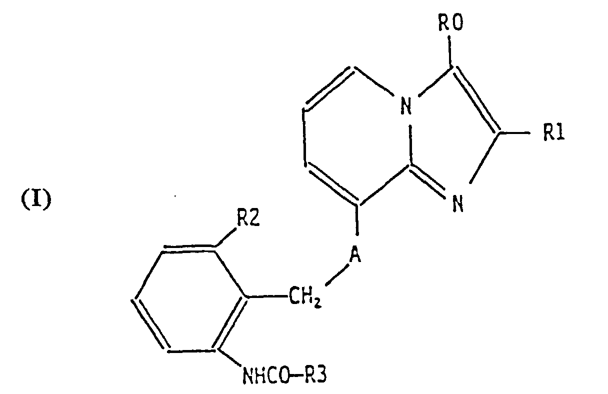 dans laquelle
R₀ représente un groupe méthyle ou hydroxyméthyle,
R₁ représente un groupe alkyle en C₁₋₄,
R₂ représente un groupe alkyle en C₁₋₄,
R₃ représente un groupe alcoxy en C₁₋₄ et
A représente un atome d'oxygène ou un groupe NH, et des sels de tels composés.

2. Composés de formule (I) selon la revendication 1, dans lesquels R₀ représente un groupe méthyle.

3. Composés de formule (I) selon la revendication 1, dans lesquels R₀ représente un groupe hydroxyméthyle.

4. Composés selon la revendication 1 choisis dans le groupe formé par
la 3-hydroxyméthyl-8-(2-méthoxycarbonylamino-6-méthylbenzylamino)-2-méthyl-imidazo[1,2-a]pyridine,
la 3-hydroxyméthyl-8-(2-méthoxycarbonylamino-6-méthylbenzyloxy)-2-méthylimidazo[1,2-a]pyridine,
la 8-(2-méthoxycarbonylamino-6-méthylbenzyloxy)-2,3-diméthylimidazo[1,2-a]pyridine,
la 8-(2-*tert*-butoxycarbonylamino-6-méthylbenzylamino)-2,3-diméthyl-imidazo[1,2-a]pyridine,
la 8-(2-*tert*-butoxycarbonylamino-6-méthylbenzyloxy)-2,3-diméthylimidazo[1,2-a]pyridine,
la 8-(2-éthoxycarbonylamino-6-méthylbenzylamino)-2,3-diméthylimidazo[1,2-a]pyridine,
la 8-(2-isobutoxycarbonylamino-6-méthylbenzylamino)-2,3-diméthylimidazo[1,2-a]pyridine,
la 8-(2-isopropoxycarbonylamino-6-méthylbenzylamino)-2,3-diméthylimidazo[1,2-a]pyridine,
la 8-(2-tert-butoxycarbonylamino-6-méthylbenzylamino)-3-hydroxyméthyl-2-méthylimidazo[1,2-a]pyridine, et
la 8-(2-tert-butoxycarbonylamino-6-méthylbenzyloxy)-3-hydroxyméthyl-2-méthylimidazo[1,2-a]pyridine,
ou un sel de ces composés.

5. Composé selon la revendication 1 appelé 8-(2-méthoxycarbonylamino-6-méthylbenzylamino)-2,3-diméthylimidazo[1,2-a]pyridine ou un sel de ce composé.

6. Procédé de préparation de composés de formule (I) selon la revendication 1, et de sels de ces composés, caractérisé par le fait
a) que l'on soumet des composés de formule (II) 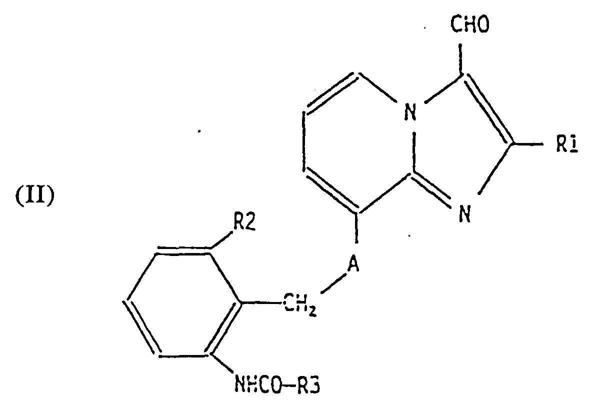 dans laquelle R₁, R₂, R₃ et A ont la signification indiquée dans la revendication 1, à une réduction de manière à obtenir des composés de formule (I) dans lesquels R₀ représente un groupe hydroxyméthyle,
b) que l'on fait réagir des composés de formule (III) 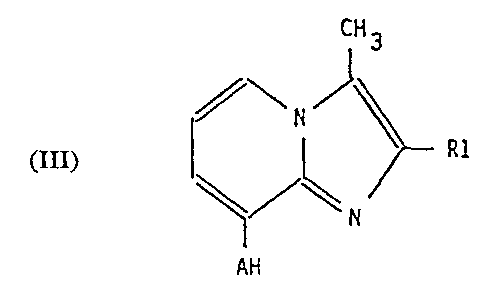 dans laquelle R₁ et A ont la signification indiquée dans la revendication 1, avec des composés de formule (IV) 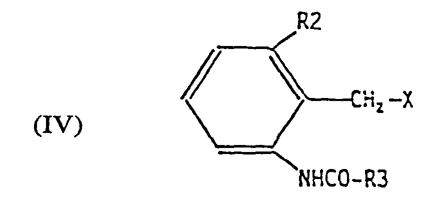 dans laquelle R₂ et R₃ ont la signification indiquée dans la revendication 1, et X représente un groupe partant approprié, de manière à obtenir des composés de formule (I) dans lesquels R₀ représente un groupe méthyle, ou
(c) que l'on fait réagir des composés de formule (V) 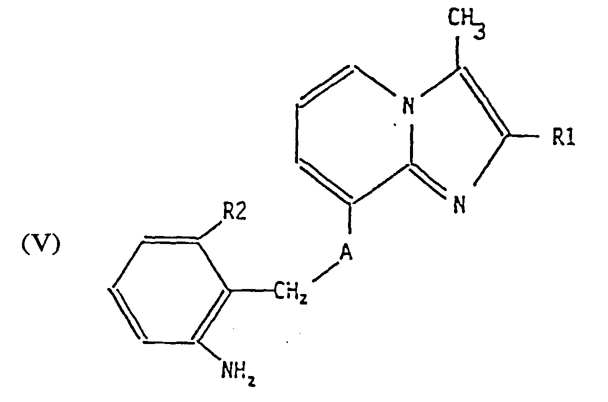 dans laquelle R¹, R² et A ont la signification indiquée dans la revendication 1, avec des composés de formule (VI)
R³-CO-Y (VI)
dans laquelle R³ a la signification indiquée dans la revendication 1 et Y représente un groupe partant approprié, de manière à obtenir des composés de formule (I) dans lesquels R⁶ représente un groupe méthyle. et que l'on convertit ensuite éventuellement les composés de formule (I) obtenus selon a), b) ou c), en leurs sels, ou que l'on convertit, si on le souhaite, les sels de composés de formule (I) obtenus en composés de formule (I).

7. Médicament contenant un composé selon la revendication 1 et/ou un sel pharmacologiquement acceptable de celui-ci.

8. Composés selon la revendication 1 et sels pharmacologiquement acceptables de ceux-ci pour la prévention et le traitement de maladies gastro-intestinales.

9. Utilisation de composés selon la revendication 1 et de sels pharmacologiquement acceptables de ceux-ci pour la fabrication de médicaments destinés à la prévention et au traitement de maladies gastrointestinales.
